Europäisches Patentamt

European Patent Office

Office européen des brevets

(11) Veröffentlichungsnummer: **0 275 363 B1**

# EUROPÄISCHE PATENTSCHRIFT

(45) Veröffentlichungstag der Patentschrift: **04.12.91**

(51) Int. Cl.⁵: **A61F 2/46**

(21) Anmeldenummer: **87114070.3**

(22) Anmeldetag: **25.09.87**

(54) **Vorrichtung zum Ausstossen oder Ansaugen flüssiger oder pastöser Medien.**

(30) Priorität: **16.01.87 DE 3701190**

(43) Veröffentlichungstag der Anmeldung:
**27.07.88 Patentblatt 88/30**

(45) Bekanntmachung des Hinweises auf die
Patenterteilung:
**04.12.91 Patentblatt 91/49**

(84) Benannte Vertragsstaaten:
**AT BE CH DE ES FR IT LI NL SE**

(56) Entgegenhaltungen:
**EP-A- 0 170 120**
**WO-A-87/05491**
**US-A- 4 024 994**

(73) Patentinhaber: **ZIEMANN, Edeltraud**
**Längenthalerstrasse 9**
**W-8252 Inning am Holz(DE)**

(72) Erfinder: **Ziemann, Edeltraud**
**Längenthalerstr. 9**
**W-8252 Inning am Holz(DE)**

(74) Vertreter: **Popp, Eugen, Dr. et al**
**MEISSNER, BOLTE & PARTNER Widenmayer-**
**strasse 48 Postfach 86 06 24**
**W-8000 München 86(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft eine Vorrichtung zum Ausstoßen oder Ansaugen flüssiger oder pastöser Medien nach dem Oberbegriff des Patentanspruches 1, wie sie beispiels weise aus der EP-A-0170 120 bekannt ist.

Um in der Gelenkchirurgie Endoprothesen mit dem Knochen fest zu verbinden, wird zunächst aus dem Knochen ein Bett herausgefräst, in dem später die Prothese mittels Knochenzement befestigt wird. Um eine einwandfreie Verbindung des Knochenzements mit dem Knochen zu erreichen, muß das Bett im wesentlichen vollständig freigespült werden von Knochenresten (vom Fräsen) oder dergleichen. Das Absaugen der Spülflüssigkeit ist relativ kritisch und darf nur mit einem geringen Unterdruck erfolgen, der darüber hinaus noch kontrollierbar sein muß.

Weiterhin muß der Knochenzement, ein "Zweikomponenten-Kunstharz" mit hoher Viskosität, in das vorbereitete Bett eingebracht werden, wobei dieser Vorgang ebenfalls kontrollierbar ablaufen muß.

Aus der CH-PS 546 075 ist eine manuell betätigbare Vorrichtung zum Einbringen von Knochenzement bekannt, die ähnlich einer handelsüblichen Injektionsspritze ausgebildet ist. Der zur Betätigung dieser Vorrichtung notwendige Kraftaufwand ist jedoch erheblich.

Es sind weiterhin Vorrichtungen im Handel, die ähnlich den Betätigungsvorrichtungen für Kunststoffkartuschen ausgebildet sind und einen schrittweisen Vorschub eines Betätigungskolbens über einer Hebel an einem Pistolengriff mit hoher Kraft ermöglichen. Diese Vorrichtungen sind aber prinzipiell nicht dazu geeignet, ein kontinuierliches Einfüllen des Knochenzements zu gewährleisten, was wiederum zu einer ungleichmäßigen und somit fehlerhaften Füllung mit Knochenzement führt.

Aus der Zeitschrift "med.-Orthop.-Techn." 6/86; Seite 203, ist eine druckluftbetätigte Vorrichtung der eingangs genannten Art bekannt, bei welcher der Kolben zum Ausstoßen des Knochenzements direkt über ein Kolben-Zylindersystem mit Druckluft beaufschlagt werden kann, so daß ein kontinuierliches und für die Bedienungsperson anstrengungsfreies Ausstoßen von Knochenzement möglich ist. Ein wesentliches Problem bei dieser Vorrichtung besteht darin, daß die Ausstoßgeschwindigkeit (Volumen pro Zeiteinheit) von Knochenzement über eine entsprechend feinfühlige Betätigung von Ventilen bewerkstelligt werden muß, welche die Zufuhr von Druckluft in den Betätigungszylinder steuern. Es hat sich nun gezeigt, daß die Bedienungsperson äußerst vorsichtig operieren muß, damit je nach momentaner (sich zeitlich ändernder) Viskosität des Knochenzements ein gleichmäßiges Ausstoßen in der gewünschten Weise erfolgt. In der bekanntermaßen oftmals hektischen Atmosphäre eines Operationssaales kann es darum relativ leicht zu "Bedienungsfehlern" mit fatalen Folgen kommen.

Ausgehend vom oben genannten Stand der Technik ist es Aufgabe der vorliegenden Erfindung, eine Vorrichtung der eingangs genannten Art dahingehend weiterzubilden, daß ein gleichmäßiges Ausstoßen mit geringem Aufwand ermöglicht wird.

Diese Aufgabe wird durch die im Kennzeichen des Patentanspruches 1 angegebenen Merkmale gelöst. Bevorzugte Ausführungsformen der Erfindung ergeben sich aus den Unteransprüchen.

Dadurch, daß die Drehung einer Druckluft-Bohrmaschine in eine lineare Verschiebebewegung umgewandelt wird, ist es möglich, eine konstante Ausstoßgeschwindigkeit sicherzustellen und zwar im wesentlichen unabhängig vom dafür notwendigen Ausstoßdruck im Zylinder. Andererseits kann dieselbe Vorrichtung zum Absaugen verwendet werden, indem man die Druckluft-Bohrmaschine im "Linkslauf" betreibt und dadurch den Kolben innerhalb des Zylinders zurückzieht.

Ein weiterer wesentlicher Vorteil der Erfindung liegt darin, daß bereits vorhandene und den besonderen Gegebenheiten im Operationssaal angepaßte Druckluft-Bohrmaschinen auf einfache Weise so umgebaut werden können, daß sowohl ein Absaugen von Spülflüssigkeit als auch ein Ausstoßen von Knochenzement in der gewünschten, präzisen Art und Weise möglich ist.

Selbstverständlich eignet sich die erfindungsgemäße Vorrichtung nicht nur zum Ausstoßen von Knochenzement, sondern auch von anderen Massen, die möglichst gleichmäßig appliziert werden sollen, wie dies z.B. bei Kontrastmitteln der Fall ist.

Vorzugsweise ist eine Begrenzungseinrichtung vorgesehen, um die Kraft, welche auf den Abtriebsschieber und damit auf den Ausstoßkolben wirkt, auf einen Maximalwert zu begrenzen. Dies bietet zum einen eine erhöhte Sicherheit gegenüber Applikation übergroßer Ausstoßdrücke, zum anderen kann damit eine Vorkomprimierung des Knochenzements mit einem definierten Druck stattfinden, durch welche sich eine verbesserte Polymerisationsreaktion erzielen läßt. Bei einer solchen Vorkomprimierung wird die Ausstoßdüse zunächst verschlossen und der Kolben in den Zylinder eingepreßt, bis die Begrenzungseinrichtung eine weitere Druckerhöhung verhindert. Wenn, wie bei einer bevorzugten Ausführungsform der Erfindung vorgesehen, das Getriebe eine von der Bohrmaschine in Drehung versetzte Gewindehülse umfaßt, welche den Abtriebsschieber über ein entsprechend korrespondierendes Außengewinde vorschiebt, so kann nach Erreichen dieses Maximaldruckes zur Vorkompression der Druck durch einfaches Abstellen der Bohrmaschine konstant gehalten werden, da das Getriebe "selbsthemmend" ist. Vor dem Öffnen der Ausstoßdüse läßt man dann die Bohrmaschine kurz im Linkslauf arbeiten, um den Druck hinreichend abzusenken.

Vorzugsweise ist der Kolben gegenüber dem Zylinder über eine Lamellendichtung abgedichtet, so daß in die Masse eingerührte Luft, wie auch bei der Reaktion entstehende Gase entweichen können, der Knochenzement mit seiner hohen Viskosität jedoch die Dichtung nicht überwinden kann. Besonders vorteilhaft ist die "Entgasungswirkung" dann, wenn im Raum hinter dem Kolben ein Unterdruck geschaffen wird. Zu diesem Zweck ist bei einer bevorzugten Ausführungsform der Erfindung im Bereich des Antriebsschiebers eine Absaugöffnung mit einem entsprechenden Sauganschluß vorgesehen.

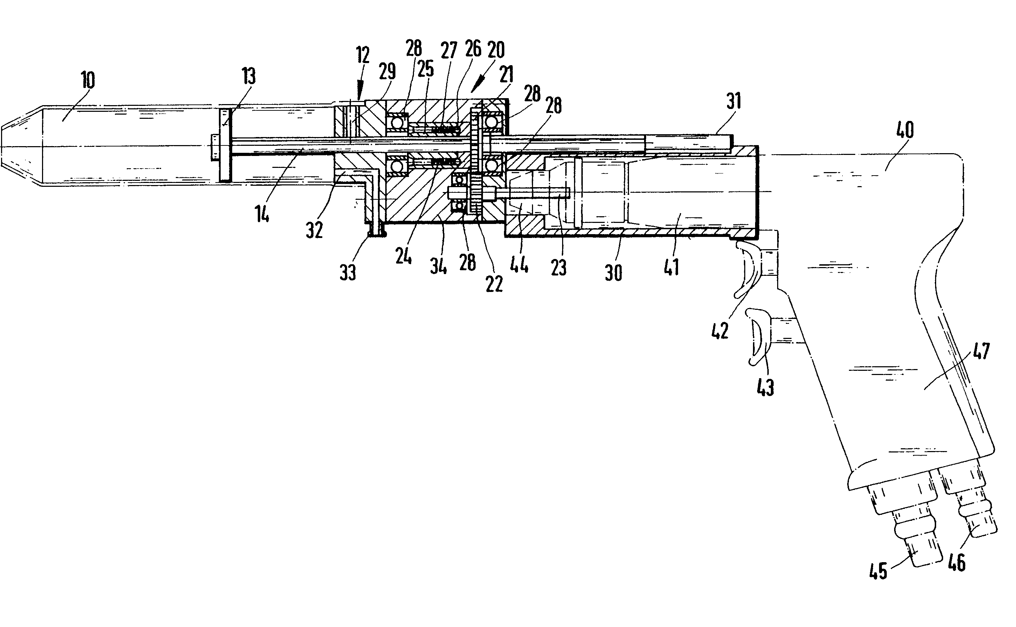

Im folgenden wir ein Ausführungsbeispiel einer bevorzugten Ausführungsform der Erfindung anhand der beiliegenden Zeichnung näher beschrieben, welche einen Teil-Längsschnitt dieser Anordnung darstellt.

Wie aus der Abbildung ersichtlich, umfaßt die erfindungsgemäße Vorrichtung eine Verschiebevorrichtung 20, die auf eine handelsübliche Druckluftbohrmaschine 40 aufsetzbar ist. Hierfür besitzt die Verschiebevorrichtung 20 einen Adapter 30, der aus einer Hülse mit distal angebrachten Spannmitteln besteht, die auf den Hals 41 der Bohrmaschine 40 formschlüssig aufsetzbar ist. Nach Anziehen der Spannmittel (nicht gezeigt) ist der Adapter 30 fest mit der Bohrmaschine 40 verbunden.

Der Adapter 30 ist mit einem Getriebegehäuse 34 fest verbunden. Im Getriebegehäuse 34 ist ein Zahnrad 22 gelagert (Kugellager 28), das auf einem Antriebszapfen 23 sitzt, welcher aus dem Gehäuse 34 hervorragt. Der Adapter 30 verläuft koaxial um den Antriebszapfen 23, der vorzugsweise eine kantige Umfangsfläche aufweist. Die Umfangsfläche des Antriebszapfens 23 ist so beschaffen, daß bei entsprechend weit geöffnetem Spannfutter 44 der Bohrmaschine 40 der Antriebszapfen 23 formschlüssig im Spannfutter 44 sitzt.

Weiterhin ist im Getriebegehäuse 34 eine zum Antriebszapfen 23 parallel aber seitlich verschobene Gewindehülse 25 drehbar aber axial unverschieblich gelagert (Kugellager 28), die mit einem weiteren Zahnrad 21, welches mit dem ersten Zahnrad 22 kämmt, über eine federbelastete Kugelkupplung mit Federn 27 und Kugeln 26 in Wirkverbindung steht. Die Anordnung bestehend aus Gewindehülse 25, Rutschkupplung 24 und Zahnrad 21 ist beidseitig gelagert, so daß eine hinreichende Stabilität gewährleistet ist.

Koaxial zur Hülse 25 weist das Gehäuse 34 eine Bohrung auf, die einen größeren Durchmesser hat als das Innengewinde der Hülse 25. In diese Bohrung des Gehäuses 34 ist ein Abtriebsschieber 14 einführbar, der ein Außengewinde aufweist, welches dem Innengewinde der Gewindehülse 25 entsprechend ausgebildet ist. Im vollständig eingesetzten Zustand sitzt also die Gewindehülse 25 auf dem Außengewinde des Abtriebsschiebers 14.

Der Abtriebsschieber 14 weist eine über seine Länge verlaufende Nut auf, in welche (im eingesetzten Zustand) ein Stift 29 inseriert, der in das Gehäuse 34 bzw. in dessen Vorderteil senkrecht zur Achse des Abtriebsschiebers 14 eingesetzt ist. Auf diese Weise ist der Abtriebsschieber 14 längsverschieblich aber verdrehsicher im Gehäuse 34 gehalten.

An seinem, der Bohrmaschine 40 abgewandten Ende ist das Gehäuse 34 mit Adaptermitteln (Bajonettkupplung) versehen, um einen Zylinder 10 mit seinem distalen Ende am Gehäuse 34 zu befestigen. Derartige Zylinder 10 sind als Einwegartikel im Handel erhältlich und müssen somit nicht genauer beschrieben werden. Am Vorderende weist der Zylinder eine Ausstoßdüse 11 auf.

Am Ende des Abtriebsschiebers 14 ist ein Kolben 13 befestigt, der eine ringsumlaufende Lamellendichtung (nicht gezeigt) aufweist.

Weiterhin ist an dem, der Bohrmaschine 40 abgewandten Ende des Gehäuses 34 eine Bohrung 32 vorgesehen, die zu einem Sauganschluß 33 führt, an den ein Schlauch ankoppelbar ist, welcher zu einer Vakuumpumpe führt. Über diesen Anschluß ist der Raum hinter dem Kolben 13 evakuierbar, wodurch eventuell im Gemisch vorhandene Lufteinschlüsse und Reaktionsgase beseitigt werden können.

Der Adapter 30 weist weiterhin auf seiner Oberseite eine Schutzkappe 31 auf, in welcher das dem Kolben 13 gegenüberliegende Ende des Abtriebsschiebers 14 laufen kann, so daß auch bei ganz zurückgezogenem Kolben 13 das Ende des Abtriebsschiebers 14 immer geschützt ist.

Die Bohrmaschine 40 ist mit zwei Ventilen versehen, über deren Betätigungsdrücker 42, 43 die Bohrmaschine 40 im Rechts- oder Linkslauf betrieben werden kann. Die Drücker 42 und 43 ragen aus einem "Pistolengriff" 47 der Bohrmaschine 40. Von der Endfläche des Pistolengriffes 47 ragen ein Druckluftanschluß 45 und ein Abluftanschluß 46, über den die meist unsterile Druckluft aus dem Operationssaal abgeführt werden kann.

Bei einer hier nicht gezeigten bevorzugten Ausführungsform der Erfindung ist anstelle des Zahnrades 22 ein Schneckenrad vorgesehen, das über eine Rutschkupplung mit dem Antriebszapfen 23 verbunden ist. Der Abtriebsschieber 14 ist bei dieser bevorzugten Ausführungsform der Erfindung nicht mit einem umlaufenden Gewinde, sondern lediglich mit Zähnen versehen, die mit dem Schnekkenrad kämmen.

Ein wesentlicher Vorteil der Erfindung liegt darin, daß eine bereits vorhandene und den Gegebenheiten des Operationssaales angepaßte Druckluftbohrmaschine 40 auf einfache Weise zur Applikatior von Knochenzement oder zum Absaugen irgendwelcher Medien verwendet werden kann, wobei die Applikation von Knochenzement besonders gut dosierbar erfolgt. Die gesamte Einrichtung ist ohne weiteres so herzustellen, daß sie dampfsterilisierbar ist, was bei elektrisch betriebenen Geräten im allgemeinen nicht der Fall ist. Darüber hinaus wird durch die Rutschkupplung eine Sicherung gegen zu hohe Ausstoßdrücke erzielt, wobei weiterhin eine Vorkompression des Knochenzementes in der gewünschten und vor allem reproduzierbaren Weise stattfinden kann.

## Patentansprüche

1. Vorrichtung zum Ausstoßen oder Ansaugen flüssiger oder pastöser (extrudierbarer) Medien im medizinisch/chirurgischen Bereich, insbesondere im Bereich der Gelenkchirurgie,

   mit einem Zylinder (10) mit distaler Ausstoßdüse (11),
   der mit seinem proximalen Ende (12) an einer Verschiebevorrichtung (20) für einen halten (13) anbringbar ist,
   **dadurch gekennzeichnet,**
   daß die Verschiebevorrichtung (20) ein Getriebe (21, 22) umfaßt, das die Drehung eines Antriebszapfens (23) in die lineare Verschiebebewegung eines Abtriebsschiebers (14) umsetzt, der mit dem Kolben (13) in Verbindung bringbar ist, und daß die Verschiebevorrichtung (20) im Bereich des Antriebszapfens (23) Adaptermittel (30) zur lösbaren Befestigung an einer Druckluft-Bohrmaschine (40) aufweist.

2. Vorrichtung nach Anspruch 1,
   **dadurch gekennzeichnet,**
   daß das Getriebe (21, 22) mit einer Begrenzungseinrichtung (24) zum Begrenzen der auf den Abtriebsschieber (14) übertragenen Kraft versehen ist.

3. Vorrichtung nach Anspruch 2,
   **dadurch gekennzeichnet,**
   daß die Begrenzungseinrichtung (24) als drehmomentbegrenzende Rutschkupplung, vorzugsweise als federbelastete Kugelkupplung (26, 27) ausgebildet ist.

4. Vorrichtung nach einem der Ansprüche 2 oder 3,
   **dadurch gekennzeichnet,**
   daß die Begrenzungseinrichtung (24) einstellbar ist.

5. Vorrichtung nach einem der vorhergehenden Ansprüche,
   **dadurch gekennzeichnet,**
   daß das Getriebe eine, in einem Getriebegehäuse (34) drehbar (durch den Antriebszapfen 23), aber unverschieblich gelagerte Gewindehülse (25) umfaßt, in welcher der mit einem korrespondierenden Außengewinde versehene Abtriebsschieber (14) sitzt, der im Getriebegehäuse (34) unverdrehbar aber längsverschieblich gelagert ist.

6. Vorrichtung nach Anspruch 5,
   **dadurch gekennzeichnet,**
   daß die Gewindehülse (25) mit einem koaxial angeordneten Zahnrad (21) in Wirkverbindung steht, das mit einem, auf dem Antriebszapfen (23) sitzenden Zahnrad (22) kämmt.

7. Vorrichtung nach Anspruch 5 und 6,
   **dadurch gekennzeichnet,**
   daß die Wirkverbindung zwischen Gewindehülse (25) und Zahnrad (21) über die Begrenzungseinrichtung (24) hergestellt ist.

8. Vorrichtung nach einem der vorhergehenden Ansprüche,
   **dadurch gekennzeichnet,**
   daß die Verschiebevorrichtung (20) im Bereich des Abtriebsschiebers (14) mit Mitteln zum lösbaren Befestigen des distalen Endes des Zylinders (10) versehen ist.

9. Vorrichtung nach einem der vorhergehenden Ansprüche,
   **dadurch gekennzeichnet,**
   daß die Verschiebevorrichtung (20) im Bereich des Abtriebsschiebers (14) mit Mitteln (32, 33) zum Absaugen von Gasen aus dem Zylinder (10) hinter dem Kolben (13) versehen ist.

10. Vorrichtung nach einem der vorhergehenden Ansprüche,
    **dadurch gekennzeichnet,**
    daß der Kolben (13) an seinem Umfang über eine Lamellendichtung zum Zylinder (10) abgedichtet ist.

## Claims

1. Device for the ejection or suction of liquid or pasty (extrudable) media in the medical/surgical field, in particular in the field of joint surgery, with a cylinder (10) with a distal ejector nozzle (11), which cylinder can be arranged with its proximal end (12) on a displacement device (20) for a piston (13), characterised in that the displacement device (20) comprises a gear (21, 22), which converts the rotation of a drive journal (23) into the linear displacement movement of a drive slide (14), which can be brought into connection with the piston (13), and in that the displacement device (20) has in the area of the drive journal (23) adapter means (30) for releasable attachment to a compressed air drill machine (40).

2. Device according to Claim 1, characterised in that the gear (21, 22) is provided with a limit arrangement (24) for limiting the force transferred to the drive slide (14).

3. Device according to Claim 2, characterised in that the limit arrangement (24) is designed as a torquelimiting slip clutch, preferably as a spring-loaded ball coupling (26, 27).

4. Device according to one of Claims 2 or 3, characterised in that the limit arrangement (24) is adjustable.

5. Device according to one of the preceding claims, characterised in that the gear comprises a threaded sleeve (25) mounted rotatably (by means of the drive journal 23) but non-displaceably in a gear housing (34), in which threaded sleeve sits the drive slide (14) provided with a corresponding external thread and mounted non-rotatably but longitudinally displaceably in the gear housing (34).

6. Device according to Claim 5, characterised in that the threaded sleeve (25) is in active connection with a coaxially arranged gearwheel (21), which meshes with a gearwheel (22) sitting on the drive journal (23).

7. Device according to Claims 5 and 6, characterised in that the active connection between threaded sleeve (25) and gearwheel (21) is obtained via the limit arrangement (24).

8. Device according to one of the preceding claims, characterised in that the displacement device (20) is provided in the area of the drive slide (14) with means for releasable attachment of the distal end of the cylinder (10).

9. Device according to one of the preceding claims, characterised in that the displacement device (20) is provided in the area of the drive slide (14) with means (32, 33) for the suctioning off of gases from the cylinder (10) behind the piston (13).

10. Device according to one of the preceding claims, characterised in that the piston (13) is sealed off around its periphery with respect to the cylinder (10) by means of a lamellar seal.

## Revendications

1. Appareil d'éjection ou de succion de matériaux fluides ou pâteux (extrudables) dans le domaine médical/chirurgical, en particulier dans le domaine de la chirurgie articulaire,

comportant un cylindre (10) à buse d'éjection distale (11) qui peut être monté, par son extrémité proximale (12), sur un dispositif de déplacement (20) d'un piston (13),

caractérisé en ce que le dispositif de déplacement (20) comprend un train d'engrenages (21,22) qui transforme la rotation d'un arbre d'entrée (23) en un mouvement de déplacement linéaire d'un coulisseau de sortie (14) qui peut être relié au piston (13) et en ce que le dispositif de déplacement (20) comprend, dans la région de l'arbre d'entrée (23), un moyen adaptateur (30) pour la fixation amovible sur une machine de perçage à air comprimé (40).

2. Appareil selon la revendication 1, caractérisé en ce que le train d'engrenages (21,22) est muni d'un dispositif de limitation (24) pour limiter la force transmise au coulisseau de sortie (14).

3. Appareil selon la revendication 2, caractérisé en ce que le dispositif de limitation (24) est réalisé sous la forme d'un accouplement à glissement, de préférence sous la forme d'un accouplement à bille (26,27) chargé par un ressort.

4. Dispositif selon l'une des revendications 2 et 3, caractérisé en ce que le dispositif de limitation (24) est réglable.

5. Dispositif selon l'une des revendications précédentes, caractérisé en ce que le train d'engrenages comprend une douille taraudée (25) montée à rotation, (par l'arbre d'entrée 23), mais non déplaçable en translation, à l'intérieur d'un carter d'engrenages (34), douille dans laquelle coopère le coulisseau de sortie (14) par un filetage externe correspondant, lequel coulisseau est monté fixe en rotation et déplaçable en translation dans le carter d'engrenages (34).

6. Dispositif selon la revendication 5, caractérisé en ce que la douille taraudée (25) est en liaison active avec une roue dentée (21) disposée coaxialement, laquelle engrène avec une roue dentée (22) calée sur l'arbre d'entrée (23).

7. Dispositif selon les revendications 5 et 6, caractérisé en ce que la liaion active entre la douille taraudée (25) et la roue dentée (21)est réalisée à travers le dispositif de limitation (24).

8. Dispositif selon l'une des revendications précédentes, caractérisé en ce que le dispositif de déplacement (20) est muni, dans la région du coulisseau de sortie (14), de moyens pour la fixation amovible de l'extrémité distale du cylindre (10).

9. Dispositif selon l'une des revendications précédentes, caractérisé en ce que le dispositif de déplacement (20) est muni, dans la région du coulisseau de sortie (14), de moyens (32,33) pour l'aspiration de gaz à partir du cylindre (10) derrière le piston (13).

10. Dispositif selon l'une des revendications précédentes, caractérisé en ce que le piston (13) est relié de façon étanche au cylindre (10), sur sa périphérie, par un joint à lamelles.